# EUROPÄISCHE PATENTSCHRIFT

(11) **EP 2 213 262 B1**
(45) Veröffentlichungstag und Bekanntmachung des Hinweises auf die Patenterteilung: **16.05.2018**
(21) Anmeldenummer: 10152067.4
(22) Anmeldetag: 29.01.2010
(51) Int. Cl.: A61F 2/38, A61F 2/30, A61F 2/00

(54) **Kniegelenkendoprothese**
Knee prosthesis
Prothèse de genou

(30) Priorität: 30.01.2009 DE 102009007724
(43) Veröffentlichungstag der Anmeldung: 04.08.2010
(73) Patentinhaber: AESCULAP AG, 78532 Tuttlingen (DE)
(72) Erfinder: Reich, Jan, 78609, Tuningen (DE); Kempf, Harry, 72510, Stetten a. k. M. (DE); Grupp, Thomas, 78588, Denkingen (DE); Miehlke, Rolf K., 48167, Münster (DE); Giurea, Alexander, 1190, Wien (AT)
(74) Vertreter: Hoeger, Stellrecht & Partner Patentanwälte mbB

(56) Entgegenhaltungen:
- EP-A1- 1 417 938
- US-A- 5 370 701
- US-A1- 2005 107 886

## Beschreibung

Die vorliegende Erfindung betrifft eine Kniegelenkendoprothese mit einer Tibiakomponente, eine Femurkomponente und einer zwischen der Tibiakomponente und der Femurkomponente an der Femur- oder Tibiakomponente beweglich gelagerten Meniskuskomponente sowie mit einer Verbindungseinrichtung zum gelenkigen Verbinden der Tibiakomponente mit der Femurkomponente und einem ersten Drehlagerelement zum rotierbaren Lagern der Femurkomponente und der Tibiakomponente relativ zueinander um eine erste Rotationsachse, wobei die Verbindungseinrichtung eine Verbindungsstellung definiert, in welcher die Tibiakomponente und die Femurkomponente unlösbar miteinander verbunden sind, wobei die Tibiakomponente eine Drehlagerelementaufnahme aufweist, in welcher das erste Drehlagerelement rotierbar gehalten ist und wobei das erste Drehlagerelement in der Drehlagerelementaufnahme in einer Richtung parallel zur ersten Rotationsachse verschiebbar gelagert ist, welche Kniegelenkendoprothese eine Anschlageinrichtung zum Begrenzen einer Bewegung des ersten Drehlagerelements und der Drehlagerelementaufnahme parallel zur ersten Rotationsachse relativ zueinander in distaler Richtung umfasst.

Kniegelenkendoprothesen mit einer Tibiakomponente, eine Femurkomponente und einer zwischen der Tibiakomponente und der Femurkomponente an der Femur- oder Tibiakomponente beweglich gelagerten Meniskuskomponente sowie mit einer Verbindungseinrichtung zum gelenkigen Verbinden der Tibiakomponente mit der Femurkomponente sind insbesondere bekannt in Form sogenannter "Scharnierprothesen", bei denen mittels eines Scharniergelenks die Flexions-/Extensionsbewegung zwischen Femur und Tibia ermöglicht wird. Eine solche Scharnierprothese ist beispielsweise aus der DE 196 18 321 C2 bekannt. Bei der in der genannten Patentschrift beschriebenen Kniegelenkendoprothese ist die Femurkomponente an der Tibiakomponente mittels eines rotierbar gelagerten Zapfens gelagert, der in eine entsprechende Aufnahme der Meniskuskomponente eingreift. Der Zapfen überträgt Querkräfte in anterior-posteriorer Richtung sowie Varus-Valgus-Momente und stabilisiert auf diese Weise das bei einem insuffizienten Bandapparat instabile Kniegelenk. Zum Verbinden der Femurkomponente mit der Tibiakomponente muss während der Implantation das Kniegelenk distrahiert werden, um die Komponenten miteinander koppeln zu können. Wird der Zapfen, wie bei der aus der DE 196 18 321 C2 bekannten Prothese in die korrespondierende Aufnahme eingeführt, ist sogar eine sehr große Distraktion erforderlich. Aufgrund der vorgeschlagenen Verbindung besteht jedoch die Gefahr, dass das Kniegelenk in Folge unbeabsichtigter Bewegungen des Patienten luxieren, also sich die Femurkomponente von der Tibiakomponente lösen kann. Eine derartige Luxation ist jedoch unerwünscht.

Eine Kniegelenkendoprothese der eingangs beschriebenen Art ist beispielsweise aus der EP 1 417 938 A1 bekannt. Weitere Kniegelenkendoprothesen sind in der US 2005/0107886 und der US 5,370,701 beschrieben.

Es ist daher eine Aufgabe der vorliegenden Erfindung, eine Kniegelenkendoprothese der eingangs beschriebenen Art so weiterzubilden, dass sie postoperativ gegen eine Luxation gesichert ist.

Diese Aufgabe wird bei einer Kniegelenkendoprothese der eingangs beschriebenen Art erfindungsgemäß dadurch gelöst, dass die Anschlageinrichtung zum Begrenzen einer Bewegung des ersten Drehlagerelements und der Drehlagerelementaufnahme relativ zueinander in proximaler Richtung ausgebildet ist. Die erfindungsgemäß vorgeschlagene Weiterbildung bekannter Kniegelenkendoprothesen stellt sicher, dass die Tibiakomponente und die Femurkomponente in der Verbindungsstellung der Verbindungseinrichtung nicht voneinander gelöst werden können. Auf diese Weise kann eine Luxation einfach und sicher verhindert werden. Insbesondere können die miteinander in Eingriff bringbaren Teile der Femurkomponente und der Tibiakomponente im Vergleich zu bisher bekannten Kniegelenkendoprothesen kürzer ausgebildet werden. Dies hat den Vorteil, dass bei der Implantation, insbesondere während des Verbindens von Femurkomponente und Tibiakomponente, keine Überdistraktion erforderlich ist und so eine bei einem geschädigten Kniegelenk ebenfalls vorgeschädigte Gelenkkapsel bzw. stabilisierende Weichteile nicht weiter verletzt werden müssen. Um die Tibiakomponente mit dem zweiten Drehlagerelement einfach und sicher verbinden zu können, ist es günstig, dass die Tibiakomponente eine Drehlagerelementaufnahme aufweist, in welcher das zweite Drehlagerelement rotierbar gehalten ist. Gemäß der Erfindung ist ein zweites Drehlagerelement zum rotierbaren Lagern der Femurkomponente und der Tibiakomponente relativ zueinander um die zweite Rotationsachse vorgesehen. Das zweite Drehlagerelement ermöglicht direkt eine Verbindung und eine Lagerung der Tibiakomponente und der Femurkomponente aneinander. Das zweite Drehlagerelement ist in der Drehlagerelementaufnahme in einer Richtung parallel zur zweiten Rotationsachse verschiebbar gelagert. Eine relative Verschiebbarkeit in einem begrenzten Bereich, das heißt über einen begrenzten Abschnitt der zweiten Rotationsachse, ermöglicht es, eine Beweglichkeit der Kniegelenkendoprothese für einen Patienten in definierter Weise zu erhöhen. Insbesondere kann so eine definierte Teilluxation zugelassen werden, das heißt ein definiertes Abheben der Femurkomponente von der Meniskuskomponente, insbesondere begrenzt bis zu einem bestimmten, zum Beispiel individuell vom Operateur vorgebbaren Abstand. In jedem Fall sind aber aufgrund der besonders gestalteten Verbindungseinrichtung die Femurkomponente von der Tibiakomponente in der Verbindungsstellung nicht lösbar voneinander, trotz der verschieblichen Lagerung relativ zueinander. Gemäß der Erfindung ist eine Anschlageinrichtung zum Begrenzen einer Bewegung des zweiten Drehlagerelements und der Drehlagerelementaufnahme relativ zueinander in distaler und/oder proximaler Richtung vorgesehen. Die Anschlageinrichtung kann insbesondere eine Bewegung der Femurkomponente und der Tibiakomponente relativ zueinander in Richtung der zweiten Rotationsachse begrenzen. Insbesondere ist es mit der Anschlageinrichtung möglich, eine definierte Luxation zuzulassen, das heißt insbesondere ein Abheben der Femurkomponente von der Meniskuskomponente beziehungsweise von der Tibiakomponente. Durch eine definierte Luxation kann ein Flexionswinkel des Kniegelenks etwas vergrößert werden. Insgesamt kann so eine einem natürlichen Kniegelenk noch ähnlichere Kniegelenkendoprothese ausgebildet werden. Durch eine Begrenzung der Bewegung des zweiten Drehlagerelements in distaler Richtung, also in Richtung auf die Tibiakomponente hin, kann insbesondere auch eine von der Femurkomponente auf die Meniskuskomponente eingeleitete Kraft durch die Anschlageinrichtung teilweise abgefangen werden. Durch die Begrenzung der Bewegung in proximaler Richtung, also in Richtung auf die Femurkomponente hin, kann eine Luxation verhindert werden.

Günstig ist es, wenn die Verbindungseinrichtung eine Montagestellung definiert, in welcher die Femurkomponente und die Tibiakomponente vollständig voneinander getrennt sind. In der Montagestellung der Verbindungseinrichtung ist es möglich, die Femurkomponente und die Tibiakomponente unabhängig voneinander an einer teilresezierten Tibia beziehungsweise einem teilresezierten Femur festzulegen, beispielsweise durch Einzementieren oder Festlegen mit Schrauben entsprechender Schäfte der beiden Komponenten in entsprechenden Aufnahmen der Knochen.

Um die Femurkomponente und die Tibiakomponente nicht bereits vor einer Implantation miteinander verbinden zu müssen, ist es vorteilhaft, wenn die Verbindungseinrichtung derart ausgebildet ist, dass sie nach einer unabhängigen Implantation der Femurkomponente und der Tibiakomponente voneinander von der Montagestellung in die Verbindungsstellung überführbar ist. Mit einer solchen Verbindungseinrichtung ist es möglich, die Femurkomponente und die Tibiakomponente miteinander zu verbinden, noch nachdem die beiden Komponenten implantiert wurden.

Vorzugsweise sind die Meniskuskomponente und die Tibiakomponente um eine zweite Rotationsachse relativ zueinander rotierbar gelagert. Eine solche Ausgestaltung ermöglicht eine Rotation insbesondere um eine von der Tibia des Patienten definierte Längsachse oder im Wesentlichen um diese Längsachse. Vorzugsweise ist jedoch eine Rotationsbewegung der Meniskuskomponente und der Tibiakomponente relativ zueinander beschränkt.

Des Weiteren ist es vorteilhaft, dass die Femurkomponente und die Tibiakomponente relativ zueinander um eine zweite Rotationsachse rotierbar gelagert sind. Beispielsweise kann die zweite Rotationsachse in Richtung oder im Wesentlichen in Richtung einer Tibialängsachse der Tibia ausgerichtet sein. Damit die Femurkomponente und die Meniskuskomponente relativ zur Tibiakomponente gemeinsam ohne Verklemmen und etwaige Reibungsverluste rotierbar sind, ist es günstig, wenn die erste und die zweite Rotationsachse identisch sind.

Vorzugsweise weist die Tibiakomponente eine Tibiakomponentengelenkfläche auf, auf welcher die Meniskuskomponente bewegbar gelagert ist. Besonders einfach lässt sich die Tibiakomponentengelenkfläche ausbilden, wenn sie eben ist. Dann ist es möglich, eine ebene oder im Wesentlichen ebene Unterseite der Meniskuskomponente auf und relativ zu der Tibiakomponentengelenkfläche zu bewegen, insbesondere zu verschieben und/oder zu rotieren.

Zur Ausbildung einer so genannten Scharnierprothese ist es vorteilhaft, wenn die Meniskuskomponente und die Femurkomponente relativ zueinander um eine erste Scharnierachse rotierbar gelagert sind. Für eine Flexions-Extensions-Bewegung der Kniegelenkendoprothese können so die Femurkomponente und die Meniskuskomponente um die erste Scharnierachse relativ zueinander verschwenkt werden.

Günstigerweise sind die Tibiakomponente und die Femurkomponente relativ zueinander um eine zweite Scharnierachse rotierbar gelagert. Eine solche Ausgestaltung ermöglicht auch eine Flexions-Extensions-Bewegung der Kniegelenkendoprothese durch Verschwenken der Tibiakomponente und der Femurkomponente relativ zueinander um die zweite Scharnierachse.

Der Aufbau der Kniegelenkendoprothese wird besonders einfach, wenn die erste und die zweite Scharnierachse identisch sind. Beispielsweise kann dies dadurch erreicht werden, dass eine Femurkomponentengelenkfläche der Femurkomponente, die an der Meniskuskomponente anliegt und an dieser abgleiten kann, konzentrisch zur zweiten Scharnierachse ausgebildet ist. Für eine optimale Führung ist eine entsprechende Gelenkfläche der Meniskuskomponente ebenfalls konzentrisch zur zweiten Scharnierachse ausgebildet, um bei einer Verschwenkbewegung einen möglichst großen Flächenkontakt zwischen den aneinander anliegenden Flächen zu erhalten.

Um die Implantation der Kniegelenkendoprothese für einen Operateur so einfach wie möglich zu machen, ist es günstig, wenn die Verbindungseinrichtung derart ausgebildet ist, dass die Tibiakomponente und die Femurkomponente nach Implantieren der Tibiakomponente an der Tibia und nach Implantieren der Femurkomponente am Femur miteinander verbindbar sind. Ein Operateur kann so insbesondere entsprechend der Physiologie des Patienten Tibiakomponente und Femurkomponente entsprechend auswählen und implantieren. Anschließend kann er nach Prüfung des Bandapparats und eines entsprechend einzuhaltenden Abstands zwischen der Tibiakomponente und der Femurkomponente ein passendes Meniskusteil auswählen, insbesondere wenn die Kniegelenkendoprothese in Form eines Kniegelenkendoprothesensatzes mit mehreren Tibiakomponenten und/oder Femurkomponenten und/oder Meniskuskomponenten bereitgestellt wird. In jedem Fall ist es ihm so möglich, die Kniegelenkendoprothese noch nach Implantation der Tibiakomponente und der Femurkomponente durch Auswahl entsprechend optimal passender Teile in Abhängigkeit der Physiologie des Patienten zu optimieren.

Günstigerweise weist die Tibiakomponente ein zweites Drehlagerelement auf zum rotierbaren Lagern der Meniskuskomponente um die zweite Rotationsachse. Beispielsweise kann das Drehlagerelement, welches in Form einer Lagerwelle ausgebildet sein kann, direkt mit einer Aufnahme, die an der Meniskuskomponente vorgesehen ist, in Eingriff gebracht werden, beispielsweise eine sacklochartige Ausnehmung oder eine Durchbrechung.

Besonders einfach wird der Aufbau der Tibiakomponente, wenn das zweite Drehlagerelement in Form eines von der Tibiakomponentengelenkfläche abstehenden Zapfens ausgebildet ist. Selbstverständlich muss der Zapfen nicht massiv ausgebildet sein. Insbesondere ist es möglich, den Zapfen hohl auszubilden, so dass er nur noch in Form einer hülsenförmigen Wand zur Ausbildung des zweiten Drehlagerelements verbleibt.

Um eine freie Rotation der Meniskuskomponente und der Tibiakomponente relativ zueinander zu verhindern, ist es günstig, wenn eine Rotationsbegrenzungseinrichtung zum Definieren eines maximalen Rotationsmittelbereich für eine Rotation der Meniskuskomponente und der Tibiakomponente relativ zueinander vorgesehen ist. Insbesondere kann der Rotationswinkelbereich ein Winkelbereich sein mit einem Wert im Bereich von 10° bis 120°. Der angegebene Rotationswinkelbereich entspricht Auslenkungen in medialer bzw. lateraler Richtung um ± 5° bis ± 60°. Vorzugsweise weist der Rotationswinkelbereich einen Wert im Bereich von 15° bis 40° auf, insbesondere kann er 24° betragen, was einer Auslenkung von ± 12° entspricht. Optional kann die Rotationsbegrenzungseinrichtung einstellbar ausgebildet sein, um den Rotationswinkelbereich individuell für jeden Patienten anpassen zu können.

Besonders einfach wird die Ausbildung der Rotationsbegrenzungseinrichtung, wenn sie einen oder zwei Drehanschläge umfasst.

Eine besonders kompakte Ausgestaltung der Kniegelenkendoprothese wird möglich, wenn der eine oder die zwei Drehanschläge am zweiten Drehlagerelement angeordnet oder ausgebildet sind. Alternativ oder zusätzlich wäre es auch denkbar, die Drehanschläge an der Meniskuskomponente auszubilden.

Vorzugsweise weist das zweite Drehlagerelement einen Querschnitt auf, welcher von einer Kreisform abweicht. Insbesondere handelt es sich bei dem beschriebenen Querschnitt um einen Schnitt parallel zur Tibiakomponentengelenkfläche. Aufgrund der von der Kreisform abweichenden Querschnittsform des zweiten Drehlagerelements können so in dieses, insbesondere durch eine entsprechende Ausgestaltung einer äußeren Oberfläche, Drehanschläge integriert werden zur Ausbildung der Rotationsbegrenzungseinrichtung. Entsprechend kann auch eine Aufnahme für das zweite Drehlagerelement an der Meniskuskomponente einen von einer Kreisform abweichenden Querschnitt aufweisen, so dass im Zusammenwirken des zweite Drehlagerelements mit der zugeordneten Aufnahme der Meniskuskomponente der Rotationswinkelbereich definiert werden kann.

Damit die Meniskuskomponente unabhängig von einer Drehstellung relativ zur Tibiakomponente stets großflächig an der Tibiakomponentengelenkfläche anliegen kann, ist es günstig, wenn die zweite Rotationsachse senkrecht zu einer von der Tibiakomponentengelenkfläche definierten Ebene orientiert ist.

Vorzugsweise sind die zweite Rotationsachse und die zweite Scharnierachse relativ zueinander windschief. Windschief bedeutet, dass sich die beiden Achsen nicht schneiden. Dies hat den Vorteil, dass sie relativ zueinander durch entsprechende Teile der Prothese beabstandet definiert werden können, um insbesondere eine möglichst natürliche Gelenkbewegung der Kniegelenkendoprothese zu ermöglichen.

Vorteilhaft ist es, wenn die zweite Rotationsachse zu einer die zweite Scharnierachse enthaltenden Ebene senkrecht steht. So können definierte Rotationsbewegungen um beide Achsen erfolgen, insbesondere dann, wenn die zweite Rotationsachse senkrecht zur Tibiakomponentengelenkfläche orientiert ist.

Um die Femurkomponente mit der Tibiakomponente in definierter Weise und sicher verbinden zu können, ist es vorteilhaft, wenn das zweite Drehlagerelement an der Tibiakomponente angeordnet oder gehalten ist. Insbesondere kann das zweite Drehlagerelement nach Implantation der Tibiakomponente mit dieser verbindbar ausgebildet und erst daran anschließend mit der Femurkomponente verbindbar sein. Auf diese Weise kann eine zur Implantation der Kniegelenkendoprothese erforderliche Distraktion des Kniegelenks minimiert werden.

Insbesondere kann das zweite Drehlagerelement rotierbar an der Tibiakomponente lagerbar sein. Vorzugsweise ist es in Form einer rotationssymmetrisch geformten Lagerwelle ausgebildet.

Vorzugsweise sind das zweite Drehlagerelement und die Tibiakomponente relativ zueinander um die zweite Rotationsachse rotierbar. Beispielsweise kann so das zweite Drehlagerelement drehfest mit der Femurkomponente verbunden werden und trotzdem noch eine Rotationsbewegung der Femurkomponente relativ zur Tibiakomponente möglich sein.

Günstig ist es, wenn die Verbindungseinrichtung derart ausgebildet ist, dass das zweite Drehlagerelement und die Tibiakomponente nach Implantieren der Tibiakomponente an der Tibia miteinander koppelbar sind. Dies gestattet es, die Tibiakomponente mit einer möglichst geringen Bauhöhe auszubilden, wodurch eine für die Implantation erforderlich Distraktion des Kniegelenks minimiert werden kann.

Zur Vermeidung einer unerwünschten Luxation ist es vorteilhaft, wenn das zweite Drehlagerelement in der Drehlagerelementaufnahme in einer Richtung parallel zur zweiten Rotationsachse unbeweglich gelagert ist. Eine Relativbewegung der Femurkomponente und der Tibiakomponente in Richtung der zweiten Rotationsachse kann sich so vollständig ausschließen lassen.

Der Aufbau der Kniegelenkendoprothese kann weiter vereinfacht werden, wenn das zweite Drehlagerelement in einer Hinterschneidung der Drehlagerelementaufnahme gehalten ist. Insbesondere ist so eine bewegliche oder unbewegliche Lagerung in axialer Richtung parallel zur zweiten Rotationsachse möglich.

Für einen besonderes kompakten Aufbau der Kniegelenkendoprothese kann es vorteilhaft sein, wenn die Anschlageinrichtung die Hinterschneidung umfasst. Insbesondere kann eine in distaler Richtung weisende Seitenfläche der Hinterschneidung, beispielsweise eine Ringfläche, eine Anschlagfläche für das zweite Drehlagerelement bilden, um eine Bewegung derselben relativ zur Tibiakomponente in proximaler Richtung zu verhindern.

Vorteilhaft ist es, wenn das zweite Drehlagerelement einen Rückhaltevorsprung umfasst, welcher in die Hinterschneidung der Drehlagerelementaufnahme eingreift. So kann das zweite Drehlagerelement einfach und sicher in der Drehlagerelementaufnahme gehalten werden, bei Bedarf auch axial verschieblich, zum Beispiel parallel zur zweiten Rotationsachse.

Damit das zweite Drehlagerelement in der Drehlagerelementaufnahme sicher gehalten werden kann, ist es günstig, wenn ein Außendurchmesser des zweiten Drehlagerelements proximalseitig des Rückhaltevorsprungs kleiner ist als ein Außendurchmesser des Rückhaltevorsprungs. So kann das zweite Drehlagerelement insbesondere proximalseitig des Rückhaltevorsprungs in der Drehlagerelementaufnahme verdrehbar geführt gehalten werden. Der Rückhaltevorsprung kann beispielsweise in eine entsprechende Hinterschneidung oder Ausnehmung des Drehlagerelements eingreifen.

Um zu verhindern, dass sich das zweite Drehlagerelement aus der Drehlagerelementaufnahme lösen kann, ist es günstig, wenn eine Sicherungseinrichtung zum Sichern des zweiten Drehlagerelements in der Drehlagerelementaufnahme vorgesehen ist.

Vorzugsweise umfasst die Sicherungseinrichtung mindestens ein erstes Sicherungselement zum Sichern des zweiten Drehlagerelements in der Drehlagerelementaufnahme. Durch das mindestens eine erste Sicherungselement kann verhindert werden, dass sich das zweite Drehlagerelement aus der Drehlagerelementaufnahme in unbeabsichtigter Weise lösen kann.

Um das Drehlagerelement in der Drehlagerelementaufnahme beziehungsweise relativ zur Tibiakomponente möglichst reibungsfrei rotierbar lagern zu können, ist vorzugsweise eine Lagerhülse zum Lagern des Drehlagerelements vorgesehen, welche in der Drehlagerelementaufnahme gehalten ist. Insbesondere kann so zwischen der Lagerhülse und dem zweiten Drehlagerelement eine Gleitpaarung mit möglichst geringen Reibungswerten ausgebildet werden, und zwar durch entsprechende Wahl insbesondere des Materials, aus dem die Lagerhülse hergestellt ist.

Besonders einfach wird der Aufbau der Kniegelenkendoprothese, wenn die Lagerhülse das erste Sicherungselement bildet. Die Lagerhülse kann insbesondere derart ausgebildet sein, dass sie einen Teil der Anschlageinrichtung bildet, um eine Bewegung des zweiten Drehlagerelements in proximaler Richtung zu begrenzen oder zu verhindern.

Vorzugweise definiert die Lagerhülse die Drehlagerelementaufnahme. Dies ermöglicht es, das zweite Drehlagerelement direkt im Inneren der Lagerhülse rotierbar und/oder axial verschiebbar zu lagern. Selbstverständlich kann an der Tibiakomponente eine Aufnahme für die Lagerhülse vorgesehen sein, die ebenfalls als Drehlagerelementaufnahme bezeichnet werden kann.

Günstigerweise weist die Tibiakomponente eine Lagerhülsenaufnahme auf zum Aufnehmen der Lagerhülse. Diese kann insbesondere in Form einer Ausnehmung an der Tibiakomponente ausgebildet sein, in die die Lagerhülse ganz oder teilweise einführbar ist. Insbesondere ist die Lagerhülsenaufnahme vorzugsweise derart ausgebildet, dass das zweite Drehlagerelement zunächst mit der Lagerhülse in Eingriff gebracht werden kann, also insbesondere in die von der Lagerhülse ausgebildete Drehlagerelementaufnahme eingesetzt werden kann, und dann die miteinander in Eingriff stehende Lagerhülse und das zweite Drehlagerelement in die Lagerhülsenaufnahme eingesetzt werden können.

Vorzugsweise umfasst die Anschlageinrichtung einen Anschlag, welcher eine Einführtiefe der Lagerhülse in die Lagerhülsenaufnahme begrenzt. Somit kann insbesondere auch verhindert werden, dass die Lagerhülse und/oder das zweite Drehlageelement an einem Boden der Lagerhülsenaufnahme anschlagen können.

Gemäß einer weiteren bevorzugten Ausführungsform der Erfindung kann vorgesehen sein, dass die Sicherungseinrichtung ein zweites Sicherungselement umfasst zum Sichern der Lagerhülse in der Lagerhülsenaufnahme. Mit dem zweiten Sicherungselement kann so die Lagerhülse in der Lagerhülsenaufnahme gesichert werden, welche Lagerhülse wiederum ein erstes Sicherungselement bilden kann zum Sichern des zweiten Drehlagerelements in der Drehlagerelementaufnahme. Insbesondere kann das zweite Sicherungselement in Form einer Überwurfmutter oder einer Sicherungshülse ausgebildet sein, die mit der Tibiakomponente verschraubbar ausgebildet sind.

Für Revisionen an der Kniegelenkendoprothese ist es vorteilhaft, wenn das zweite Sicherungselement mit der Tibiakomponente lösbar verbindbar ist. Dies gestattet es, die Lagerhülse bei Bedarf aus der Lagerhülsenaufnahme entnehmen zu können.

Vorzugsweise ist das zweite Sicherungselement mit der Lagerhülsenaufnahme lösbar verbindbar. So kann es bei entsprechender Ausbildung möglichst direkt mit der Lagerhülse zusammenwirken und diese in der Lagerhülsenaufnahme halten.

Falls keine Lagerhülse vorgesehen sein sollte, kann es ferner günstig sein, wenn das zweite Sicherungselement mit der Drehlagerelementaufnahme lösbar verbindbar ist. Das zweite Sicherungselement kann in einem solchen Fall das zweite Drehlagerelement direkt in der Drehlagerelementaufnahme gegen eine Axialbewegung sichern oder eine solche begrenzen.

Besonders einfach und kostengünstig herstellen lässt sich die Lagerhülse aus einem Kunststoff.

Eine Reibung zwischen einem insbesondere aus einem Implantatstahl hergestellten zweiten Drehlagerelement und der Lagerhülse kann minimiert werden, wenn der Kunststoff Polyetheretherketon (PEEK) ist oder enthält.

Um die Stabilität der Lagerhülse zu erhöhen, ist es vorteilhaft, wenn der Kunststoff ein faserverstärkter Kunststoff ist.

Damit ferner eine Gleitpaarung zwischen der Meniskuskomponente und einer aus einem Implantatstahl hergestellten Femurkomponente optimiert werden kann, ist es vorteilhaft, wenn die Meniskuskomponente aus einem Kunststoff hergestellt ist.

Besonders langlebige Meniskusteile lassen sich herstellen und so lange Standzeiten erreichen, wenn der Kunststoff Polyethylen (PE) ist. Insbesondere kann es sich um Polyethylen mit einem hohen Molekulargewicht oder um hochverdichtetes Polyethylen handeln.

Vorzugsweise ist das zweite Drehlagerelement von proximal her kommend in die Drehlagerelementaufnahme einsetzbar. Dies ermöglicht es, das zweite Drehlagerelement in Abhängigkeit einer Physiologie des Patienten auszuwählen und nach endgültiger Implantation der Tibiakomponente an der Tibia mit der Tibiakomponente in Eingriff zu bringen.

Gemäß einer weiteren bevorzugten Ausführungsform der Erfindung kann eine Kupplungseinrichtung zum Koppeln des zweiten Drehlagerelements mit der Femurkomponente vorgesehen sein. Dabei können das zweite Drehlagerelement und die Femurkomponente direkt oder indirekt miteinander verbunden werden.

Eine besonders einfache Konstruktion der Kupplungseinrichtung ist möglich, wenn sie ein Kupplungsteil umfasst, wenn das Drehlagerelement über das Kupplungsteil indirekt mit der Femurkomponente verbindbar ist und wenn das Kupplungsteil und die Femurkomponente relativ zueinander um die zweite Scharnierachse rotierbar gelagert sind. Mittels des Kupplungsteils ist eine indirekte Verbindung zwischen dem zweiten Drehlagerelement und der Femurkomponente möglich. Ferner kann über die Ausgestaltung des Kupplungsteils auch eine Orientierung der zweiten Rotationsachse relativ zur zweiten Scharnierachse definiert werden, insbesondere ob diese sich gegenseitig schneiden oder windschief zueinander stehen.

Um die Stabilität der Kniegelenkendoprothese zu verbessern, ist es günstig, wenn das zweite Drehlagerelement und das Kupplungsteil relativ zueinander unbeweglich aneinander festlegbar sind. Auf diese Weise kann ein Materialabrieb zwischen dem zweiten Drehlagerelement und dem Kupplungsteil vermieden oder zumindest minimiert werden.

Besonders einfach verbinden lassen sich das Drehlagerelement und die Femurkomponente miteinander, wenn die Kupplungseinrichtung eine Schraubverbindung umfasst zum Verbinden des zweiten Drehlagerelements und der Femurkomponente miteinander.

Vorzugsweise ist die Schraubverbindung ausgebildet zwischen dem zweiten Drehlagerelement und dem Kupplungsteil. Auf diese Weise kann das Drehlagerelement insbesondere axial und drehfest mit dem Kupplungsteil verbunden werden.

Günstig ist es, wenn das Kupplungsteil eine Kupplungshülse umfasst und wenn das zweite Drehlagerelement in der Kupplungshülse gehalten ist. Die Kupplungshülse kann das zweite Drehlagerelement definiert positionieren, wodurch auch die Stabilität der Verbindung zwischen dem zweiten Drehlagerelement und dem Kupplungsteil erhöht werden kann.

Vorzugsweise sind das zweite Drehlagerelement und das Kupplungsteil klemmend miteinander verbunden. Eine klemmende Verbindung kann alternativ oder zusätzlich zu einer Schraubverbindung vorgesehen sein, um eine möglichst dauerhafte und spielfreie Verbindung zwischen dem zweiten Drehlagerelement und dem Kupplungsteil herzustellen.

Eine Klemmung zwischen dem zweiten Drehlagerelement und dem Kupplungsteil lässt sich auf besonders einfache Weise dadurch erreichen, dass das zweite Drehlagerelement einen in der Kupplungshülse klemmend gehaltenen Konusabschnitt umfasst. Vorzugsweise ist eine Innenwand der Kupplungshülse entsprechend dem Konusabschnitt konisch geformt, um eine möglichst flächige Konusklemmung zu erreichen.

Grundsätzlich wäre es möglich, das zweite Drehlagerelement und die Kupplungshülse allein über einen Klemmsitz aneinander zu sichern und miteinander zu verbinden. Günstig ist es, wenn die Schraubverbindung das zweite Drehlagerelement in der Kupplungshülse sichert. So kann insbesondere ein Lösen des Kupplungsteils vom zweiten Drehlagerelement verhindert werden sowohl zum einen optional durch eine Klemmverbindung und zum anderen durch eine Schraubverbindung.

Die Herstellung und die Konstruktion der Kniegelenkendoprothese lassen sich weiter vereinfachen, wenn die Schraubverbindung einen am zweiten Drehlagerelement ausgebildeten Außengewindeabschnitt und eine zum Außengewindeabschnitt korrespondierende Sicherungsmutter umfasst. Insbesondere wenn der Außengewindeabschnitt an einer Seite aus der Kupplungshülse vorsteht, kann er mit der Sicherungsmutter verschraubt werden, insbesondere bis diese an der Kupplungshülse anschlägt.

Um eine Bewegung des zweiten Drehlagerelements in proximaler Richtung zu begrenzen, ist es vorteilhaft, wenn die Kupplungshülse einen proximalen Anschlag für das zweite Drehlagerelement bildet. Sie dient also einerseits zum Verbinden mit dem zweiten Drehlagerelement und andererseits zur Bewegungsbegrenzung in proximaler Richtung, das heißt in Richtung auf die Femurkomponente hin.

Das zweite Drehlagerelement, welches einen proximalen Endabschnitt mit einem Außengewinde aufweisen kann, kann auf einfache Weise mit einer Mutter verschraubt werden, wenn ein proximales Ende des zweiten Drehlagerelements proximalseitig mit dem Außengewindeabschnitt aus der Kupplungshülse vorsteht oder die Kupplungshülse eine Aufnahme für die Mutter bildet.

Gemäß einer weiteren bevorzugten Ausführungsform der Erfindung kann vorgesehen sein, dass die Femurkomponente zwei Kondylenabschnitte umfasst, welche an der Meniskuskomponente anliegende Femurkomponentengelenkflächen definieren. Eine derart ausgestaltete Femurkomponente ist in ihrem konstruktiven Aufbau einem natürlichen Femur mit seinen Kondylen angenähert.

Ein besonders kompakter Aufbau der Kniegelenkendoprothese kann insbesondere dadurch erreicht werden, dass das Kupplungsteil im Bereich zwischen den Kondylenabschnitten gelenkig mit der Femurkomponente gekoppelt ist. Dies ermöglicht es insbesondere, die Kupplungseinrichtung, insbesondere das Kupplungsteil, so anzuordnen, dass es nicht über eine von den Femurkomponentengelenkflächen definierte Hüllfläche vorsteht.

Um insbesondere eine Austreten des zweiten Drehlagerelements in distaler Richtung aus der Drehlagerelementaufnahme zu verhindern, ist es günstig, wenn diese distalseitig geschlossen ist. Ferner kann sie so auch einen in distaler Richtung wirksamen Anschlag zur Begrenzung einer Bewegung des zweiten Drehlagerelements in distaler Richtung bilden.

Damit das zweite Drehlagerelement auf einfache Weise in die Drehlagerelementaufnahme eingesetzt werden kann, insbesondere nach Implantation der Tibiakomponente an der Tibia des Patienten, ist es vorteilhaft, wenn die Drehlagerelementaufnahme proximalseitig offen ist.

Die Herstellung insbesondere der Tibiakomponente kann weiter vereinfacht werden, wenn die Drehlagerelementaufnahme sacklochartig ausgebildet ist. Die Herstellung eines Sacklochs ist in der Regel einfach zu realisieren. Ein Sackloch, insbesondere eine derartig ausgebildete Drehlagerelementaufnahme, kann zudem unterschiedliche Abschnitte parallel zur zweiten Rotationsachse aufweisen, die sich in ihren Innendurchmessern unterscheiden.

Um die Stabilität der Kniegelenkendoprothese nach der Implantation zu optimieren, ist es günstig, wenn sich die Drehlagerelementaufnahme mindestens teilweise oberhalb und/oder unterhalb der Tibiakomponentengelenkfläche erstreckt. Auf diese Weise lassen sich eingeleitete Querkräfte optimal abfangen.

Um noch während einer Implantation der Kniegelenkendoprothese eine perfekte Anpassung an die Anatomie des Patienten zu ermöglichen, ist es vorteilhaft, wenn die Kniegelenkendoprothese mindestens zwei unterschiedlich lange zweite Drehlagerelemente zur Ausbildung eines modularen Kniegelenkendoprothesensatzes umfasst. Es können auch drei, vier, fünf oder noch mehr zweite Drehlagerelemente vorgesehen sein. In Abhängigkeit des erforderlichen Abstands zwischen der Femurkomponente und der Tibiakomponente kann ein Operateur nach Implantation der beiden Komponenten die erforderliche Höhe der Meniskuskomponente ermitteln, eine passende Meniskuskomponente auswählen, die erforderliche Länge des zweiten Drehlagerelements ermitteln, das am besten passende zweite Drehlagerelement auswählen und mit der Tibiakomponente verbinden.

Gemäß einer weiteren bevorzugten Ausführungsform der Erfindung kann ferner vorgesehen sein, dass die Kniegelenkendoprothese mindestens zwei unterschiedlich geformte Meniskuskomponenten und/oder Femurkomponenten und/oder Tibiakomponenten zur Ausbildung eines modularen Kniegelenkendoprothesensatzes umfasst. Es können auch drei, vier, fünf oder noch mehr unterschiedliche Varianten der genannten Komponenten vorgesehen sein, um eine optimierte Auswahl derselben in Abhängigkeit der Anatomie des Patienten treffen zu können.

Die nachfolgende Beschreibung bevorzugter Ausführungsformen der Erfindung dient im Zusammenhang mit der Zeichnung der näheren Erläuterung. Es zeigen:
- Figur 1:: eine Explosionsdarstellung einer Kniegelenksendoprothese;
- Figur 2:: eine Längsschnittansicht der in Figur 1 dargestellten Kniegelenkendoprothese in der Verbindungsstellung; und
- Figur 3:: eine vergrößerte Ansicht des Bereichs A aus Figur 2.

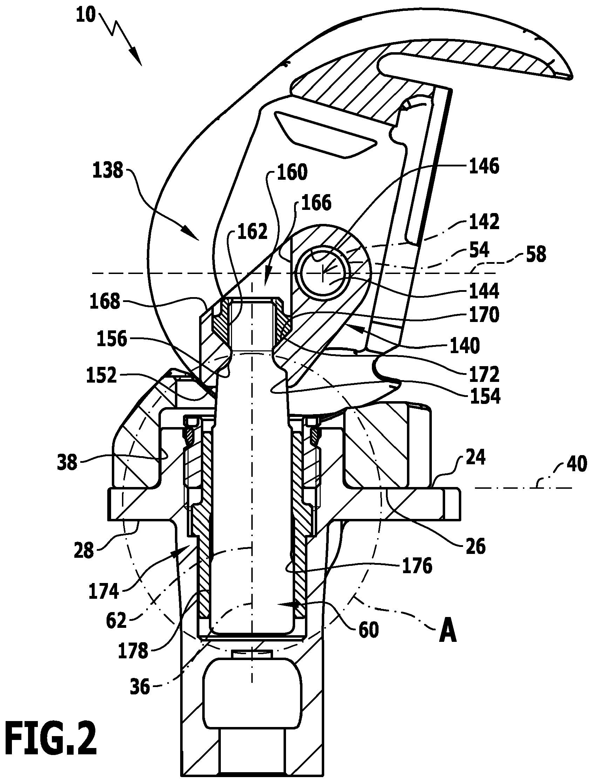

In den Figuren ist eine insgesamt mit dem Bezugszeichen 10 bezeichnete Kniegelenkendoprothese dargestellt. Sie umfasst eine an einem Femur eines Patienten implantierbare Femurkomponente 12, eine an einer Tibia implantierbare Tibiakomponente 14 sowie eine zwischen diesen angeordnete und beweglich gelagerte Meniskuskomponente 16. Ferner umfasst die Kniegelenkendoprothese 10, die in Form einer Scharnierprothese ausgebildet ist, eine Verbindungseinrichtung 18, welche eine Verbindungsstellung definiert, in welcher die Tibiakomponente 14 und die Femurkomponente 12 unlösbar miteinander verbunden sind, wie insbesondere in Figur 2 dargestellt und nachfolgend näher erläutert wird.

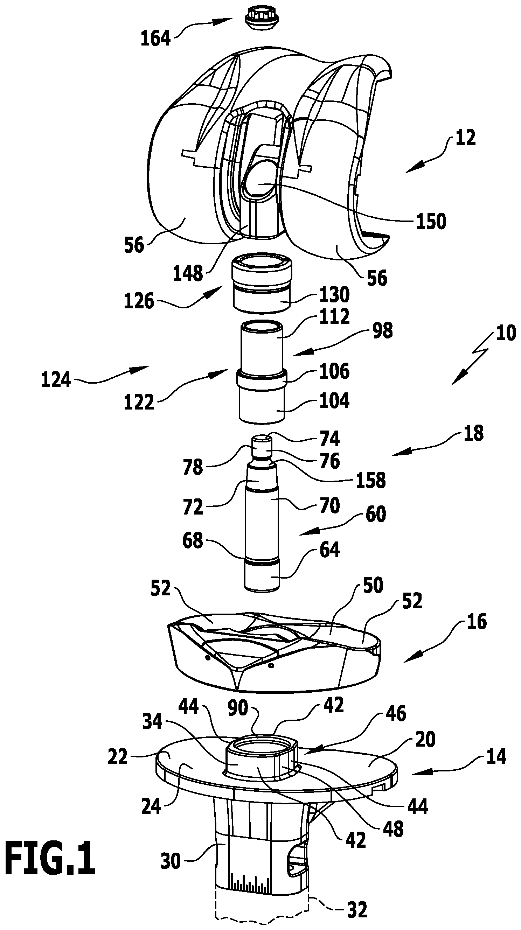

In einer Montagestellung der Verbindungseinrichtung 18 sind, wie in Figur 1 dargestellt, die Femurkomponente 12 und die Tibiakomponente 14 vollständig voneinander getrennt. Die Femurkomponente 12 kann so unabhängig von der Tibiakomponente 14 mit dem Femur verbunden werden, beispielsweise mittels Knochenzement und/oder durch Verschrauben. Ebenso kann die Tibiakomponente 14 unabhängig von der Femurkomponente 12 mit der Tibia verbunden werden, vorzugsweise ebenfalls durch Zementieren und/oder Verschrauben.

Die Tibiakomponente 14 umfasst eine Gelenkplatte 20, welche eine ebene, in proximaler Richtung weisende Oberfläche 22 aufweist, welche eine Tibiakomponentengelenkfläche 24 definiert, auf welcher eine ebene Unterseite 26 der Meniskuskomponente 16 flächig anliegt. Distalseitig ist an einer Unterseite 28 der Tibiakomponente 14 ein kurzer Schaftabschnitt 30 angeformt, welcher mit einem Tibiaschaft 32 variabler Länge verbindbar ist, und zwar in der in der DE 20 2008 903 817 U1 beschriebenen Weise.

Von der Tibiakomponentengelenkfläche 24 erstreckt sich in proximaler Richtung ein erstes Drehlagerelement 34 in Form eines flachen Kupplungszapfens. Es definiert eine erste Rotationsachse 36 und kann im Wesentlichen formschlüssig in eine korrespondierende Aufnahme 38, welche in Form einer Durchbrechung der Meniskuskomponente 16 ausgebildet ist, eingeführt werden. Die erste Rotationsachse 36 ist senkrecht zu einer durch die Oberfläche 22 definierten Tibiaebene 40 ausgerichtet.

Das erste Drehlagerelement 34 weist zwei von der ersten Rotationsachse 36 weg weisende und konzentrisch zu dieser angeordnete, einander diametral gegenüberliegende Zylinderflächenbereiche 42 auf, die sich jeweils über einen Winkelbereich von etwa 80° erstrecken und in anteriorer beziehungsweise posteriorer Richtung weisen. Zwischen den Zylinderflächenbereichen 42 sind abgeflachte, mehrere ebene Flächenabschnitte umfassende Seitenflächen 44 ausgebildet, die einen Teil einer Rotationsbegrenzungseinrichtung 46 bilden zum Begrenzen einer Rotation der Meniskuskomponente 16 um das erste Drehlagerelement 34. Mit den Seitenflächen 44 zusammenwirken korrespondierende, in den Figuren nicht dargestellte Vorsprünge, welche an der Aufnahme 38 ausgebildet sind und in Richtung auf die erste Rotationsachse 36 hin weisen. Die Seitenflächen 44 bilden somit Anschläge 48 für die an der Aufnahme 38 vorgesehenen Vorsprünge. Es kann so ein Rotationswinkelbereich um die erste Rotationsachse definiert werden, mit welchem beispielsweise ein Rotationswinkel in einem Bereich von 10° bis 120° vorgebbar ist, je nach Ausgestaltung der Seitenflächen 44 in Verbindung mit den an der Aufnahme 38 ausgebildeten Vorsprüngen. Der angegebene Rotationswinkelbereich entspricht Auslenkungen in medialer bzw. lateraler Richtung um ± 5° bis ± 60°. Vorzugsweise weist der Rotationswinkelbereich einen Wert im Bereich von 15° bis 40° auf, insbesondere kann er 24° betragen, was einer Auslenkung von ± 12° entspricht.

Auf einer Oberseite 50 der Meniskuskomponente 16, welche spiegelsymmetrisch zu einer die erste Rotationsachse 36 enthaltenden Spiegelebene ausgebildet ist, welche der in Figur 2 dargestellten Schnittebene entspricht, sind zwei Gelenkflächen 52 ausgebildet, und zwar konzentrisch zu einer ersten Scharnierachse 54. Die Femurkomponente 12 umfasst zwei ebenfalls spiegelsymmetrisch zu einer die erste Rotationsachse 36 enthaltenden Spiegelebene, die der Schnittebene in Figur 2 entspricht, ausgebildete Kondylenbereiche oder -abschnitte 56, die mindestens abschnittsweise konzentrisch zur ersten Scharnierachse 54 geformt und mit den Gelenkflächen 52 flächig in Anlage bringbar sind. Die Meniskuskomponente 16 und die Femurkomponente 12 können so relativ zueinander um die erste Scharnierachse 54 verschwenkt werden. Die erste Rotationsachse 36 ist senkrecht zu einer die erste Scharnierachse 54 enthaltenden Ebene 58 orientiert, von dieser beabstandet und somit relativ zur ersten Scharnierachse 54 windschief ausgerichtet.

Die Verbindungseinrichtung 18 umfasst ferner ein zweites Drehlagerelement 60, welches an der Tibiakomponente 14 rotierbar um eine zweite Rotationsachse 62 rotierbar gelagert ist, die mit der ersten Rotationsachse 36 identisch ist. Das zweite Drehlagerelement 60 ist in Form eines rotationssymmetrischen, eine Lagerwelle definierenden Bolzens ausgebildet, welcher insgesamt vier Abschnitte aufweist, nämlich einen distalen Zylinderabschnitt 64, der sich ausgehend von einem distalen Ende 66 in proximaler Richtung erstreckt und einen maximalen Außendurchmesser des zweiten Drehlagerelements 60 definiert. Proximalseitig schließt sich über eine abgeschrägte Schulter 68 verbunden an den Zylinderabschnitt 64 ein ebenfalls zylindrischer Führungsabschnitt 70 an, welcher einen etwas kleineren Außendurchmesser aufweist als der Zylinderabschnitt 64, jedoch etwa doppelt so lang in Richtung der ersten Rotationsachse 36 ist wie der Zylinderabschnitt 64. Proximalseitig schließt sich an den Führungsabschnitt 70 ein schwach konischer Konusabschnitt 72 an, welcher etwas kürzer ist als der Zylinderabschnitt 64. Ein proximales Ende 74 des zweiten Drehlagerelements 60 bildet ein kurzer Gewindebolzenabschnitt 76, welcher mit einem Außengewinde 78 versehen ist.

Das zweite Drehlagerelement 60 ist in einer an der Tibiakomponente 14 ausgebildeten Drehlagerelementaufnahme 80 rotierbar sowie parallel zur zweiten Rotationsachse 62 in einem begrenzten Umfang verschiebbar gelagert. Die Drehlagerelementaufnahme 80 ist in Form eines Sacklochs ausgebildet, welches in Richtung auf die Femurkomponente 12 hin, also proximalseitig, geöffnet ist. In distaler Richtung ist die Drehlagerelementaufnahme 80 geschlossen, wobei ein Boden 82 des Sacklochs einen in distaler Richtung wirkenden Anschlag definiert. Die Drehlagerelementaufnahme 80 erstreckt sich durch das erste Drehlagerelement 34 hindurch, teilweise in den Schaftabschnitt 30 hinein und somit teilweise proximalseitig und teilweise distalseitig der Tibiaebene 40.

Ausgehend vom Boden 82 erstreckt sich ein erster hohlzylindrischer Abschnitt 84 der Drehlagerelementaufnahme 80 in proximaler Richtung. Er erweitert sich einstufig im Außendurchmesser zu einem zweiten hohlzylindrischen Abschnitt 86 hin, so dass eine parallel zur Tibiaebene 40 in proximaler Richtung weisende Ringfläche 88 ausgebildet wird. Etwas beabstandet von der Ringfläche 88 bis fast zu einem proximalen Ende 90 der Drehlagerelementaufnahme 80, welcher definiert wird durch eine in proximaler Richtung weisende Randfläche 92 des ersten Drehlagerelements 34, erstreckt sich ein in einer Innenfläche 94 des zweiten Abschnitts 86 ausgebildeter Innengewindeabschnitt 96.

Das zweite Drehlagerelement 60 ist nicht direkt in der Drehlagerelementaufnahme 80 gehalten, sondern in einer Lagerhülse 98. Sie definiert quasi eine Drehlagerelementaufnahme 102 für das zweite Drehlagerelement 60. Die Drehlagerelementaufnahme 80 definiert so eine Lagerhülsenaufnahme 100 für die Lagerhülse 98. Die Lagerhülse 98 ist etwas kürzer als ein Abstand zwischen dem Boden 82 und der Randfläche 92. Ein distaler Abschnitt 104 weist einen Außendurchmesser auf, welcher dem Innendurchmesser des ersten Abschnitts 84 entspricht. An den Abschnitt 104 schließt sich proximalseitig ein in radialer Richtung nach außen abstehender Ringvorsprung 106 an, welcher eine in distaler Richtung weisende Ringfläche 108 definiert, die an der Ringfläche 88 anliegt. Eine in distaler Richtung weisende Stirnrandfläche 110 ist etwas vom Boden 82 beabstandet. Ein sich an den Ringvorsprung 106 proximalseitig anschließender Hülsenabschnitt 112 weist einen Außendurchmesser auf, welcher etwas kleiner ist als ein Außendurchmesser des Abschnitts 104.

Die Lagerhülse 98 ist im Inneren hohlzylindrisch und weist einen distalen Abschnitt 114 auf, mit einem Innendurchmesser, der dem Außendurchmesser des Zylinderabschnitts 64 entspricht. Der distale Abschnitt 114 geht über eine etwas in distaler Richtung weisende Schulter 116 in einen proximalen Abschnitt 118 über, dessen Innendurchmesser dem Außendurchmesser des Führungsabschnitts 70 entspricht. Ist das zweite Drehlagerelement 60 maximal weit in Richtung auf den Boden 82 vorgeschoben, sind die Schultern 68 und 116 voneinander beabstandet und definieren einen maximalen Abstand 120 voneinander. Dieser Abstand 120 entspricht auch dem maximalen Weg, den das zweite Drehlagerelement 60 relativ zur Lagerhülse 98 verschiebbar ist. Ausgehend von der distalsten Stellung des zweiten Drehlagerelements 60 in der Drehlagerelementaufnahme 102 kann das zweite Drehlagerelement 60 in proximaler Richtung, dass heißt in Richtung auf die Femurkomponente 12 hin, um maximal einen Weg verschoben werden, der dem Abstand 120 entspricht. Dadurch, dass die Schultern 68 und 116 in Anlage kommen, begrenzen sie eine weitere Bewegung des zweiten Drehlagerelements 60 relativ zur Drehlagerelementaufnahme 102.

Die Lagerhülse 98 definiert ein erstes Sicherungselement 122 zum Sichern des zweiten Drehlagerelements 60 in der Drehlagerelementaufnahme 80 und bildet einen Teil einer insgesamt mit dem Bezugszeichen 124 bezeichneten Sicherungseinrichtung zum Sichern des zweiten Drehlagerelements 60 in der Drehlagerelementaufnahme 80.

Die Sicherungseinrichtung 124 umfasst ferner ein zweites Sicherungselement 126 zum Sichern der Lagerhülse 98 in der Drehlagerelementaufnahme 80 beziehungsweise der Lagerhülsenaufnahme 100. Das zweite Sicherungselement 126 ist in Form einer Sicherungshülse ausgebildet, welche einen Außengewindeabschnitt 130 umfasst, der mit einem Außengewinde versehen ist, welches zum Innengewindeabschnitt 96 korrespondiert, und welcher Außengewindeabschnitt 130 sich ausgehend von einem distalen Ende 128 des zweiten Sicherungselements 126 in proximaler Richtung über etwa zwei Drittel einer Gesamtlänge des zweiten Sicherungselements 126 erstreckt. An den Außengewindeabschnitt 130 schließt sich proximalseitig eine in radialer Richtung von der zweiten Rotationsachse 62 weg weisend geöffnete Ringnut 132 an, in die ein Sicherungsreifen 134 eingesetzt ist. Der Sicherungsreifen 134 ist vorzugsweise aus einem Kunststoff, beispielsweise aus Polyethylen, hergestellt und verhindert ein unbeabsichtigtes Lösen des zweiten Sicherungselements 126, wenn dieses mit seinem Außengewindeabschnitt 130 mit dem Innengewindeabschnitt 96 verschraubt ist.

Das Ende 128 ist etwas beabstandet von einer in proximaler Richtung weisenden ringförmigen Anschlagfläche 136, welche durch den Ringvorsprung 106 definiert wird. Dies ermöglicht zudem eine Bewegung der Lagerhülse 98 in der Lagerhülsenaufnahme 100 derart, dass in einer distalsten Stellung der Lagerhülse 98 die Ringfläche 108 an der Ringfläche 88 anliegt, in einer proximalsten Stellung der Lagerhülse 98 in der Drehlagerelementaufnahme 80 die Anschlagfläche 136 am Ende 128.

Zum Verbinden des zweiten Drehlagerelements 60 mit der Femurkomponente 12 dient eine insgesamt mit dem Bezugszeichen 138 bezeichnete Kupplungseinrichtung. Sie umfasst ein Kupplungsteil 140, welches das zweite Drehlagerelement 60 indirekt mit der Femurkomponente 12 verbindet, so dass das Kupplungsteil 140 und die Femurkomponente 12 um eine zweite Scharnierachse 142 verschwenkbar relativ zueinander gelagert sind. Die zweite Scharnierachse 142 wird definiert durch eine Längsachse eines Lagerbolzens 144, welche eine Bohrung 146 durchsetzt und fest mit der Femurkomponente 12 verbunden ist in einem Bereich zwischen den Kondylenbereichen 56.

Das Kupplungsteil 140 definiert einen Hülsenabschnitt 148, welcher von einer rotationssymmetrisch geformten Durchbrechung 150 durchsetzt ist. Ausgehend von einem distalen Ende 152 schließt sich an dieses im Innern ein kurzer Konusabschnitt 154 an, welcher korrespondierend zum Konusabschnitt 72 ausgebildet ist, um das Kupplungsteil 140 und das zweite Drehlagerelement 60 klemmend miteinander zu verbinden. An den Konusabschnitt 154 angrenzend verjüngt sich einen Innendurchmesser der Durchbrechung 150 entsprechend einer einen Übergangsbereich 158 zwischen dem Konusabschnitt 72 und dem Gewindeabschnitt 76 definierenden Außendurchmesserverringerung des zweiten Drehlagerelements 60, so dass ein so definierter Übergangsbereich 156 der Durchbrechung 150 flächig am Übergangsbereich 158 zwischen dem Konusabschnitt 72 und dem Gewindebolzenabschnitt 76 anliegt. Durch die Gestaltung der Übergangsbereiche 156 und 158 bildet der Hülsenabschnitt 148 des Kupplungsteils 140 einen proximalen Anschlag für das zweite Drehlagerelement 60.

Eine Schraubverbindung 160 ist ausgebildet zum Sichern einer Verbindung zwischen dem zweiten Drehlagerelement 60 und dem Kupplungsteil 140. Der Kupplungsteil 140 umfasst eine Kupplungshülse in Form des Hülsenabschnitts 148, in welcher der Konusabschnitt 72 sowie der Übergangsbereich 158 klemmend gehalten sind. Die Schraubverbindung 160 dient zum Sichern des zweiten Drehlagerelements 60 im Hülsenabschnitt 148. Sie umfasst den Gewindebolzenabschnitt 76 und eine ein korrespondierend zum Außengewinde 78 ausgebildetes Innengewinde 162 aufweisende Sicherungsmutter 164.

Der Gewindebolzenabschnitt 76 steht proximalseitig über den Übergangsbereich 156 vor, ragt jedoch nicht über ein proximales Ende 168 des Hülsenabschnitts 148 hinaus. An den Übergangsbereich 156 schließt sich ein sich konisch erweiternder Abschnitt 170 an, gegen den ein distales, entsprechend konisch geformtes Ende 172 der Sicherungsmutter 164 gespannt werden kann. An den Abschnitt 170 schließt sich eine hohlzylindrische Erweiterung 166 an, welche die Sicherungsmutter 164 vollständig aufnimmt.

Die Funktionsweise sowie die Implantation der Kniegelenkendoprothese 10 mit der einen Luxationssicherungsmechanismus bildenden Verbindungseinrichtung 18 wird nachfolgend beschrieben.

Zunächst werden Knochenlager in Femur und Tibia des Patienten präpariert und anschließend die Femurkomponente 12 und die Tibiakomponente 14 entsprechend eingesetzt. Dies kann mit oder ohne Knochenzement erfolgen.

In einem nächsten Schritt wird die Meniskuskomponente 16 auf die Tibiakomponente 14 gesetzt, wobei das erste Drehlagerelement 34, welches einen von einer Kreisform abweichenden Querschnitt aufweist, in die Aufnahme 38 eingreift. Danach wird das zweite Drehlagerelement 60 von distal her kommend mit dem Gewindebolzenabschnitt 76 voran in die Lagerhülse 98 eingeschoben und danach beide zusammen in die Drehlagerelementaufnahme 80 beziehungsweise die Lagerhülsenaufnahme 100 eingesetzt.

Anschließend wird das zweite Sicherungselement 126 von proximal her kommend über das zweite Drehlagerelement 60 geschoben und mit der Lagerhülsenaufnahme 100 verschraubt und fest angezogen. Das Kupplungsteil 140 wird dann über das zweite Drehlagerelement 60 von proximal her kommend gestülpt. Hierfür müssen Femur und Tibia nur minimal distrahiert werden. Mittels der Sicherungsmutter 164 werden das zweite Drehlagerelement 60 und das Kupplungsteil 140 der Femurkomponente 12 miteinander gekoppelt.

Zweite Drehlagerelement 60, entsprechende Lagerhülsen 98 sowie zweite Sicherungselemente 126 stehen optional entsprechend verfügbarer Höhen von Meniskuskomponenten 16 zur Verfügung, so dass jeweils die maximale Führungslänge für das zweite Drehlagerelement 60 ausgenutzt werden kann. Es wird so für einen Operateur insgesamt ein Kniegelenkendoprothesensatz bereitgestellt, aus dem er die am besten passenden Teile für den jeweiligen Patienten auswählen und miteinander zur Kniegelenkendoprothese 10 zusammenfügen kann.

Der besondere Aufbau der Verbindungseinrichtung 18 ermöglicht somit eine rotierbare Lagerung der Tibiakomponente 14 und der Femurkomponente 12 relativ zueinander um die zweite Scharnierachse 142 und um die zweite Rotationsachse 62. Die Verbindungseinrichtung 18 stellt zudem sicher, dass das zweite Drehlagerelement 16 nicht luxieren kann. Dies bedeutet, dass es aufgrund von parallel zur Rotationsachse 62 wirkenden Kräften weder von der Femurkomponente 12 noch von der Tibiakomponente 14 getrennt werden kann. Durch Bereitstellen entsprechender zweiter Drehlagerelemente 60, zweiter Sicherungselemente 128 und Lagerhülsen 98 können unterschiedliche Meniskuskomponenten 16 mit variablen Höhen genutzt werden, so dass die Verbindungseinrichtung 18 "mitwachsen" kann, das heißt sie kann die jeweils zur Verfügung stehende maximale Führungslänge für das zweite Drehlagerelement 60 ausnutzen.

Des Weiteren erlaubt die besondere Ausgestaltung der Verbindungseinrichtung 18 eine begrenzte Subluxation, nämlich das oben beschriebene Axialspiel des zweiten Drehlagerelements 60 in der Drehlagerelementaufnahme 102. Dies wird erreicht durch eine Anschlageinrichtung 174 zum Begrenzen der Bewegung des zweiten Drehlagerelements 60 und der Drehlagerelementaufnahme 80 relativ zueinander in distaler und proximaler Richtung. Die Anschlageinrichtung 174 umfasst eine Hinterschneidung 176 der Lagerhülse 98, welche gebildet wird durch den distalen Abschnitt 114, der im Innendurchmesser im Vergleich zum proximalen Abschnitt 118 erweitert ist. Der Zylinderabschnitt 64 selbst definiert dann einen Rückhaltevorsprung 178, welcher in die Hinterschneidung 176 eingreift. Die Anschlageinrichtung 174 umfasst Anschläge, die gebildet werden durch die Schultern 116 und 68, die in einer proximalsten Stellung des zweiten Drehlagerelements 60 aneinander anliegen. In distaler Richtung wird eine Bewegung des zweiten Drehlagerelements 60 begrenzt durch den Boden 82.

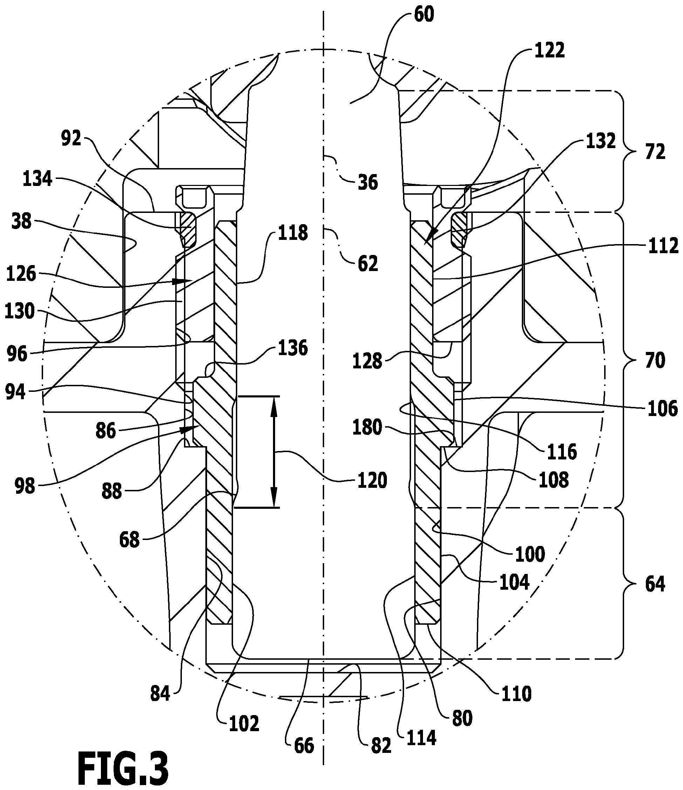

Bei der beschriebenen Ausführungsform der Kniegelenkendoprothese 10 umfasst die Anschlageinrichtung 174 ferner den Ringvorsprung 106, welcher distalseitig an die Ringfläche 88 in Anlage bringbar ist und nach entsprechender Verschiebung der Lagerhülse 98 in proximaler Richtung an das distale Ende 128 des zweiten Sicherungselements 126. Die Ringfläche 88 bildet so einen Anschlag 180, welcher eine Einführtiefe der Lagerhülse 98 in die Lagerhülsenaufnahme 100 begrenzt. Dadurch, dass der Anschlag 180 und das Ende 128 etwas voneinander beabstandet sind, kann somit eine Art Pumpbewegung ermöglicht werden zwischen dem zweiten Drehlagerelement 60 und der Tibiakomponente 14. In Abhängigkeit der wirkenden Reibungskräfte zwischen dem zweiten Drehlagerelement 60, der Lagerhülse 98 und der Tibiakomponente 14 kann beispielsweise ausgehend von einer distalsten Stellung sowohl des zweiten Drehlagerelements 60 als auch der Lagerhülse 98, wie sie in den Figuren 2 und 3 dargestellt ist, bei auf das zweite Drehlagerelement 60 wirkenden Zugkräften zum Beispiel dieses zunächst relativ zur Lagerhülse 98 bewegt werden, bis die Schultern 68 und 116 aneinander anschlagen. Weiterhin in proximaler Richtung auf das zweite Drehlagerelement 60 wirkende Kräfte führen dann noch zu einer Bewegung der Lagerhülse 98 in der Lagerhülsenaufnahme 100, bis die Anschlagfläche 136 am Ende 128 anschlägt. In umgekehrter Richtung würde dann eine entsprechend analoge, teleskopartige Bewegung erfolgen.

Die Lagerhülse 98 ist zur Ausbildung einer möglichst reibungsarmen Gleitpaarung aus einem Kunststoff hergestellt. Der Kunststoff enthält vorzugsweise Polyetheretherketon (PEEK) oder ist Polyetheretherketon. Ferner kann der Kunststoff faserverstärkt sein, um eine Stabilität der Lagerhülse 98 zu verbessern.

Abgesehen von der Meniskuskomponente 16, der Lagerhülse 98 sowie des Sicherungsreifens 134 sind alle übrigen Teile der Kniegelenkendoprothese 10 körperverträglichen Metall, beispielsweise aus einem Implantatstahl oder aus Titan hergestellt. Die Meniskuskomponente 16 ist vorzugsweise aus einem Kunststoff, beispielsweise aus Polyethylen (PE) hergestellt. Insbesondere kann es sich dabei um Polyethylen mit einem hohen Molekulargewicht oder um hochdichtes Polyethylen handeln.

## Patentansprüche

1. Kniegelenkendoprothese (10) mit einer Tibiakomponente (14), einer Femurkomponente (12) und einer zwischen der Tibiakomponente (14) und der Femurkomponente (12) an der Femur- oder Tibiakomponente (12, 14) beweglich gelagerten Meniskuskomponente (16) sowie mit einer Verbindungseinrichtung (18) zum gelenkigen Verbinden der Tibiakomponente (14) mit der Femurkomponente (12) und einem ersten Drehlagerelement (60) zum rotierbaren Lagern der Femurkomponente (12) und der Tibiakomponente (14) relativ zueinander um eine erste Rotationsachse (62), wobei die Verbindungseinrichtung (18) eine Verbindungsstellung definiert, in welcher die Tibiakomponente (14) und die Femurkomponente (12) unlösbar miteinander verbunden sind, wobei die Tibiakomponente (14) eine Drehlagerelementaufnahme (80) aufweist, in welcher das erste Drehlagerelement (60) rotierbar gehalten ist und wobei das erste Drehlagerelement (60) in der Drehlagerelementaufnahme (80) in einer Richtung parallel zur ersten Rotationsachse (62) verschiebbar gelagert ist, welche Kniegelenkendoprothese (10) eine Anschlageinrichtung (174) zum Begrenzen einer Bewegung des ersten Drehlagerelements (60) und der Drehlagerelementaufnahme (80) parallel zur ersten Rotationsachse (62) relativ zueinander in distaler Richtung umfasst, wobei die Anschlageinrichtung (174) zum Begrenzen einer Bewegung des ersten Drehlagerelements (60) und der Drehlagerelementaufnahme (80) parallel zur ersten Rotationsachse (62) relativ zueinander in proximaler Richtung ausgebildet ist.

2. Kniegelenkendoprothese nach Anspruch 1, **dadurch gekennzeichnet, dass** die Verbindungseinrichtung (18) eine Montagestellung definiert, in welcher die Femurkomponente (12) und die Tibiakomponente (14) vollständig voneinander getrennt sind.

3. Kniegelenkendoprothese nach Anspruch 1 oder 2, **dadurch gekennzeichnet, dass** die Verbindungseinrichtung (18) derart ausgebildet ist, dass sie nach einer unabhängigen Implantation der Femurkomponente (12) und der Tibiakomponente (14) voneinander von der Montagestellung in die Verbindungsstellung überführbar ist.

4. Kniegelenkendoprothese nach einem der voranstehenden Ansprüche, **dadurch gekennzeichnet, dass** die Meniskuskomponente (16) und die Tibiakomponente (14) um eine zweite Rotationsachse (36) relativ zueinander rotierbar gelagert sind.

5. Kniegelenkendoprothese nach einem der voranstehenden Ansprüche, **dadurch gekennzeichnet, dass** die Meniskuskomponente (16) und die Femurkomponente (12) relativ zueinander um eine erste Scharnierachse (54) rotierbar gelagert sind.

6. Kniegelenkendoprothese nach einem der voranstehenden Ansprüche, **dadurch gekennzeichnet, dass** die Verbindungseinrichtung (18) derart ausgebildet ist, dass die Tibiakomponente (14) und die Femurkomponente (12) nach Implantieren der Tibiakomponente (14) an der Tibia und nach Implantieren der Femurkomponente (12) am Femur miteinander verbindbar sind.

7. Kniegelenkendoprothese nach einem der voranstehenden Ansprüche, **dadurch gekennzeichnet, dass** die Tibiakomponente (14) ein zweites Drehlagerelement (34) aufweist zum rotierbaren Lagern der Meniskuskomponente (16) um die zweite Rotationsachse (36).

8. Kniegelenkendoprothese nach einem der voranstehenden Ansprüche, **gekennzeichnet durch** eine Rotationsbegrenzungseinrichtung (46) zum Definieren eines maximalen Rotationswinkelbereichs für eine Rotation der Meniskuskomponente (16) und der Tibiakomponente (14) relativ zueinander.

9. Kniegelenkendoprothese nach einem der voranstehenden Ansprüche, **gekennzeichnet durch** eine Lagerhülse (98) zum Lagern des Drehlagerelements (60), wobei die Lagerhülse (98) in der Drehlagerelementaufnahme (80) gehalten ist und wobei die Lagerhülse (98) eine weitere Drehlagerelementaufnahme (102) definiert.

10. Kniegelenkendoprothese nach Anspruch 9, **dadurch gekennzeichnet, dass** das erste Drehlagerelement (60) in einer Hinterschneidung (176) der weiteren Drehlagerelementaufnahme (102) gehalten ist.

11. Kniegelenkendoprothese nach Anspruch 10, **dadurch gekennzeichnet, dass** die Anschlageinrichtung (174) die Hinterschneidung (176) umfasst.

12. Kniegelenkendoprothese nach einem der Ansprüche 9 bis 11, **dadurch gekennzeichnet, dass** die Tibiakomponente (14) eine Lagerhülsenaufnahme (100) aufweist zum Aufnehmen der Lagerhülse (98) und dass die Anschlageinrichtung (174) einen Anschlag (180) umfasst, welcher eine Einführtiefe der Lagerhülse (98) in die Lagerhülsenaufnahme (100) begrenzt.

13. Kniegelenkendoprothese nach einem der voranstehenden Ansprüche, **gekennzeichnet durch** eine Kupplungseinrichtung (138) zum Koppeln des ersten Drehlagerelements (60) mit der Femurkomponente (12).

14. Kniegelenkendoprothese nach einem der voranstehenden Ansprüche, **gekennzeichnet durch** mindestens zwei unterschiedlich lange erste Drehlagerelemente (60) zur Ausbildung eines modularen Kniegelenkendoprothesensatzes.

15. Kniegelenkendoprothese nach einem der voranstehenden Ansprüche, **gekennzeichnet durch** mindestens zwei unterschiedlich geformte Meniskuskomponenten (16) und/oder Femurkomponenten (12) und/oder Tibiakomponenten (14) zur Ausbildung eines modularen Kniegelenkendoprothesensatzes.

## Claims

1. Knee joint endoprosthesis (10) having a tibial component (14), a fermoral component (12), and a meniscal component (16) which is moveably mounted on the femoral or tibial component (12, 14) between the tibial component (14) and the femoral component (12), and having a connecting device (18) for connecting the tibial component (14) to the femoral component (12) in an articulated manner, and a first pivot bearing element (60) for mounting the femoral component (12) and the tibial component (14) so as to be rotatable relative to each other about a first rotational axis (62), wherein the connecting device (18) defines a connecting position in which the tibial component (14) and the femoral component (12) are nondetachably connected to each other, wherein the tibial component (14) has a pivot bearing element receiver (80) in which the first pivot bearing element (60) is rotatably held, and wherein the first pivot bearing element (60) is mounted in the pivot bearing element receiver (80) so as to be displaceable in a direction parallel to the first rotational axis (62), which knee joint endoprosthesis (10) comprises a stop device (174) for delimiting a movement of the first pivot bearing element (60) and the pivot bearing element receiver (80) in parallel to the first rotational axis (62) relative to each other in distal direction, wherein the stop device (174) is configured for delimiting a movement of the first pivot bearing element (60) and the pivot bearing element receiver (80) in parallel to the first rotational axis (62) relative to each other in proximal direction.

2. Knee joint endoprosthesis according to Claim 1, **characterized in that** the connecting device (18) defines an assembly position in which the femoral component (12) and the tibial component (14) are completely separated from each other.

3. Knee joint endoprosthesis according to either one of Claims 1 or 2, **characterized in that** the connecting device (18) is configured in such a way that it is transferable from the assembly position into the connecting position, after an implantation of the femoral component (12) and the tibial component (14) independently from each other.

4. Knee joint endoprosthesis according to any one of the preceding Claims, **characterized in that** the meniscal component (16) and the tibial component (14) are mounted so as to be rotatable relative to each other about a second rotational axis (36).

5. Knee joint endoprosthesis according to any one of the preceding Claims, **characterized in that** the meniscal component (16) and the femoral component (12) are mounted so as to be rotatable relative to each other about a first hinge axis (54).

6. Knee joint endoprosthesis according to any one of the preceding Claims, **characterized in that** the connecting device (18) is configured in such a way that the tibial component (14) and the femoral component (12) are connectable to each other, after implanting the tibial component (14) to the tibia, and after implanting the femoral component (12) to the femur.

7. Knee joint endoprosthesis according to any one of the preceding Claims, **characterized in that** the tibial component (14) has a second pivot bearing element (34) for mounting the meniscal component (16) so as to be rotatable about the second rotational axis (36).

8. Knee joint endoprosthesis according to any one of the preceding Claims, **characterized by** a rotation delimiting device (46) for defining a maximum rotation angle range for a rotation of the meniscal component (16) and the tibial component (14) relative to each other.

9. Knee joint endoprosthesis according to any one of the preceding Claims, **characterized by** a bearing sleeve (98) for mounting the pivot bearing element (60), wherein the bearing sleeve (98) is held in the pivot bearing element receiver (80), and wherein the bearing sleeve (98) defines a further pivot bearing element receiver (102).

10. Knee joint endoprosthesis according to Claim 9, **characterized in that** the first pivot bearing element (60) is held in an undercut (176) of the further pivot bearing element receiver (102).

11. Knee joint endoprosthesis according to Claim 10, **characterized in that** the stop device (174) comprises the undercut (176).

12. Knee joint endoprosthesis according to any one of Claims 9 to 11, **characterized in that** the tibial component (14) has a bearing sleeve receiver (100) for receiving the bearing sleeve (98), and **in that** the stop device (174) comprises a stop (180) which delimits an insertion depth of the bearing sleeve (98) into the bearing sleeve receiver (100).

13. Knee joint endoprosthesis according to any one of the preceding Claims, **characterized by** a coupling device (138) for coupling the first pivot bearing element (60) to the femoral component (12).

14. Knee joint endoprosthesis according to any one of the preceding Claims, **characterized by** at least two first pivot bearing elements (60) of different length for forming a modular knee joint endoprosthesis set.

15. Knee joint endoprosthesis according to any one of the preceding Claims, **characterized by** at least two differently shaped meniscal components (16) and/or femoral components (12) and/or tibial components (14) for forming a modular knee joint endoprosthesis set.

## Revendications

1. Endoprothèse de l'articulation du genou (10) avec un composant tibia (14), un composant fémur (12) et un composant ménisque (16) monté de manière mobile entre le composant tibia (14) et le composant fémur (12) sur le composant fémur ou tibia (12, 14) ainsi qu'avec un dispositif de liaison (18) pour la liaison articulée du composant tibia (14) avec le composant fémur (12) et un premier élément de palier rotatif (60) pour le montage du composant fémur (12) et du composant tibia (14) de manière à pouvoir tourner l'un par rapport à l'autre autour d'un premier axe de rotation (62), où le dispositif de liaison (18) définit une position de liaison dans laquelle le composant tibia (14) et le composant fémur (12) sont reliés entre eux de manière inamovible, où le composant tibia (14) présente un logement d'élément de palier rotatif (80) dans lequel le premier élément de palier rotatif (60) est maintenu de manière à pouvoir tourner et où le premier élément de palier rotatif (60) est monté de manière déplaçable dans le logement d'élément de palier rotatif (80) dans une direction parallèle au premier axe de rotation (62), laquelle endoprothèse de l'articulation du genou (10) comprend un dispositif de butée (174) pour la limitation d'un mouvement du premier élément de palier rotatif (60) et du logement d'élément de palier rotatif (80) parallèlement au premier axe de rotation (62) l'un par rapport à l'autre en direction distale, où le dispositif de butée (174) est agencé pour la limitation d'un mouvement du premier élément de palier rotatif (60) et du logement d'élément de palier rotatif (80) parallèlement au premier axe de rotation (62) l'un par rapport à l'autre en direction proximale.

2. Endoprothèse de l'articulation du genou selon la revendication 1, **caractérisée en ce que** le dispositif de liaison (18) définit une position de montage dans laquelle le composant fémur (12) et le composant tibia (14) sont totalement séparés l'un de l'autre.

3. Endoprothèse de l'articulation du genou selon la revendication 1 ou 2, **caractérisée en ce que** le dispositif de liaison (18) est agencé de telle manière qu'il peut être amené à passer de la position de montage à la position de liaison après une implantation du composant fémur (12) et du composant tibia (14) indépendamment l'un de l'autre.

4. Endoprothèse de l'articulation du genou selon l'une des revendications précédentes, **caractérisée en ce que** le composant ménisque (16) et le composant tibia (14) sont montés de manière à pouvoir tourner l'un par rapport à l'autre autour d'un second axe de rotation (36).

5. Endoprothèse de l'articulation du genou selon l'une des revendications précédentes, **caractérisée en ce que** le composant ménisque (16) et le composant fémur (12) sont montés de manière à pouvoir tourner l'un par rapport à l'autre autour d'un premier axe de charnière (54).

6. Endoprothèse de l'articulation du genou selon l'une des revendications précédentes, **caractérisée en ce que** le dispositif de liaison (18) est agencé de telle manière que le composant tibia (14) et le composant fémur (12) peuvent être reliés l'un à l'autre après l'implantation du composant tibia (14) sur le tibia et après l'implantation du composant fémur (12) sur le fémur.

7. Endoprothèse de l'articulation du genou selon l'une des revendications précédentes, **caractérisée en ce que** le composant tibia (14) présente un second élément de palier rotatif (34) pour le montage du composant ménisque (16) de manière à pouvoir tourner autour du second axe de rotation (36).

8. Endoprothèse de l'articulation du genou selon l'une des revendications précédentes, **caractérisée par** un dispositif de limitation de rotation (46) pour la définition d'un domaine d'angle de rotation maximal pour une rotation du composant ménisque (16) et du composant tibia (14) l'un par rapport à l'autre.

9. Endoprothèse de l'articulation du genou selon l'une des revendications précédentes, **caractérisée par** une douille de palier (98) pour le montage de l'élément de palier rotatif (60), où la douille de palier (98) est maintenue dans le logement d'élément de palier rotatif (80) et où la douille de palier (98) définit un autre logement d'élément de palier rotatif (102).

10. Endoprothèse de l'articulation du genou selon la revendication 9, **caractérisée en ce que** le premier élément de palier rotatif (60) est maintenu dans une contre-dépouille (176) de l'autre logement d'élément de palier rotatif (102).

11. Endoprothèse de l'articulation du genou selon la revendication 10, **caractérisée en ce que** le dispositif de butée (174) comprend la contre-dépouille (176).

12. Endoprothèse de l'articulation du genou selon l'une des revendications 9 à 11, **caractérisée en ce que** le composant tibia (14) présente un logement de douille de palier (100) pour le logement de la douille de palier (98) et **en ce que** le dispositif de butée (174) comprend une butée (180) qui limite une profondeur d'insertion de la douille de palier (98) dans le logement de douille de palier (100).

13. Endoprothèse de l'articulation du genou selon l'une des revendications précédentes, **caractérisée par** un dispositif de couplage (138) pour le couplage du premier élément de palier rotatif (60) avec le composant fémur (12).

14. Endoprothèse de l'articulation du genou selon l'une des revendications précédentes, **caractérisée par** au moins deux premiers éléments de palier rotatif (60) de longueur différente pour la formation d'un ensemble d'endoprothèse de l'articulation du genou modulaire.

15. Endoprothèse de l'articulation du genou selon l'une des revendications précédentes, **caractérisée par** au moins deux composants ménisque (16) et/ou composants fémur (12) et/ou composants tibia (14) formés de manière différente pour la formation d'un ensemble d'endoprothèse de l'articulation du genou modulaire.
